# FASCICULE DE BREVET EUROPEEN

(11) **EP 0 836 838 B1**
(45) Date de publication et mention de la délivrance du brevet: **11.06.2003**
(21) Numéro de dépôt: 97203143.9
(22) Date de dépôt: 09.10.1997
(51) Int. Cl.: A61F 2/00

(54) **Prothèse anatomique pour la réparation d'hernies par voie laparoscopique ou ouverte**
Anatomische Prothese für Reparatur von Inguinalhernien (Leistenbrüchen) durch offene oder laparoskopische Chirurgie
Anatomical prosthesis for inguinal hernia repair via open of laparoscopic surgery

(30) Priorité: 18.10.1996 FR 9612932
(43) Date de publication de la demande: 22.04.1998
(73) Titulaire: SOFRADIM PRODUCTION, 01600 Trévoux (FR)
(72) Inventeur: Benchetrit, Salomon, 69300 Caluire (FR)
(74) Mandataire: Guerre, Dominique

(56) Documents cités:
- EP-A- 0 719 527
- WO-A-92/06639
- US-A- 4 769 038
- US-A- 5 147 374

## Description

La présente invention concerne une prothèse anatomique pour la réparation d'hernies, et en particulier une prothèse adaptée à la réparation d'hernies inguinales par laparoscopie.

De manière générale, les prothèses pour la réparation de la région inguinale et pour le traitement d'hernies sont bien connues, et sont constituées notamment d'une ou plusieurs plaques prothétiques, poreuses, en matière synthétique biocompatible, résorbable ou non, par exemple, en polyéthylène, polypropylène, polyester, ou similaire, éventuellement ayant subi un traitement de surface pour rendre celle-ci biocompatible avec le milieu cellulaire dans lequel elles sont implantées.

Ces prothèses peuvent être obtenues, par exemple, en assemblant par tricotage deux plaques de matériau prothétique. Une telle prothèse a été décrite dans le brevet américain US-A-4 769 038, délivré à Bendavid et al, celle-ci étant conçue essentiellement pour la reconstruction complète de la région inguinale. Conformément à ce brevet, la prothèse comprend une première plaque, supérieure, sensiblement plane, et une deuxième plaque, inférieure, lesdites plaques comprenant chacune un matériau prothétique poreux et flexible. Ces plaques de forme généralement allongée sont reliées entre elles selon une ligne droite de liaison par un moyen de liaison, par exemple une couture. La prothèse comprend en outre une troisième plaque, reliée aux deux autres selon la même ligne droite de liaison, et par la même couture.
EP-A-0 719 527 décrit une prothèse pour la réparation des hernies comprenant trois plaques reliées entre elles par un renfort selon une ligne droite de liaison commune.

Lorsqu'on effectue une réparation d'hernie dans la région inguinale par voie laparoscopique postérieure et extrapéritonéale, il est très important de pouvoir reconnaître, et recouvrir avec la prothèse, certains éléments anatomiques de la paroi abdominale antérieure, que l'on peut décrire comme suit, de dedans en dehors, et pour le côté droit du corps :
- en dedans, l'espace rétro-pariétal antérieur est limité en avant par les muscles grands droits, en arrière par le péritoine, et en bas par le bord supérieur du pubis ;
- la partie moyenne est limitée en avant par le fascia transversalis, et le tendon conjoint, en bas on trouve les vaisseaux iliaques, et en haut le muscle transverse ;
- dans la partie externe, on trouve en avant l'orifice interne du canal inguinal avec les éléments du cordon (vaisseaux spermatiques et canal déférent), en bas le muscle psoas, et en haut le muscle transverse.

En résumant, et ceci constitue le point de départ de la présente invention, on remarque que la région inguinale est particulière en ce que les éléments décrits ci-dessus ne sont pas tous dans le même plan dans l'espace, mais disposés de manière oblique de haut en bas, et de dehors en dedans. En cas d'hernie inguinale, la prothèse mise en place après réduction de l'hernie doit assurer une bonne couverture en s'adaptant aux reliefs de la région et en respectant l'obliquité de l'espace inguinal, sans laisser subsister de vides si possible.

Cette nécessité pose un problème important lors de l'utilisation des prothèses connues, car aucune n'est vraiment adaptée à l'anatomie du site de l'intervention chirurgicale, ce qui oblige le chirurgien à découper la prothèse, souvent à plusieurs reprises, afin d'obtenir une prothèse plus ou moins bien conformée. Or, une mauvaise couverture des éléments anatomiques décrits ci-dessus est probablement l'une des causes principales des récidives d' hernies, de telles hernies pouvant être encore plus difficiles à traiter du fait de la dégradation des structures anatomiques provoquée par l'hernie antérieure.

La présente invention a donc pour objet une prothèse anatomique sans découpe importante et préalable, et plus particulièrement destinée à la réparation de hernies inguinales par voie laparoscopique postérieure et extrapéritonéale, en assurant une bonne couverture de l'espace inguinal, sans pour autant préformer (par exemple par thermoformage) le matériau constitutif et souple de chaque plaque.

La prothèse selon l'invention se différencie des prothèses connues en ce qu'elle comporte deux plaques asymétriques l'une par rapport à l'autre, et dans la conformation déployée de la prothèse, la deuxième plaque présente au moins une forme développée ondulée, et anatomique pour épouser la forme générale des structures inguinales inférieures, notamment les vaisseaux spermatiques et iliaques, et le muscle psoas, et en correspondance la ligne de liaison présente au moins une forme courbe ondulée, la génératrice décrivant ladite forme développée et passant par la ligne de liaison étant dirigée selon un angle d'ouverture θ au plus égal à 150° par rapport au plan de la première plaque.

Une prothèse telle que précédemment définie assure une couverture de tous les éléments anatomiques précédemment décrits, sans laisser de vides susceptibles d'être la cause d'une récidive. En particulier, la région autour des vaisseaux iliaques et spermatiques est particulièrement bien protégée.

Par ailleurs, cette prothèse offre un confort accru pour le sujet dans lequel elle est implantée, car contrairement aux prothèses connues, elle ne nécessite que peu ou pas d'agrafes de fixation. La prothèse selon la présente invention reste d'elle-même en place, car la ligne de liaison se positionne à l'intersection des plans pariétaux et vasculaires. Ceci permet à la prothèse de suivre les changements de position relative des différents éléments anatomiques de la région inguinale, qui résultent du mouvement normal des muscles abdominaux du sujet, sans toutefois se déplacer en dehors de la région d'implantation. Par comparaison, les prothèses connues et telles que décrites dans le brevet américain précité, sont agrafées ou fixées par sutures aux structures abdominales environnantes, donc sous tension, et un mouvement musculaire banal, par exemple, le fait de tousser, ou se lever d'une position assise, provoque souvent une sensation désagréable, voire des douleurs.

Par ailleurs, une prothèse selon l'invention peut être aisément introduite et implantée dans l'espace inguinal, selon les techniques usuelles laparoscopiques ou ouvertes. La ligne de liaison, incorporant le moyen de liaison (couture par exemple), à la fois flexible selon sa longueur, mais relativement rigide, joue le rôle d'un longeron donnant l'axe de pose de la prothèse, tout en jouant le rôle d'une charnière pour son déploiement.

Conformément à un mode d'exécution préférentiel de la prothèse de la présente invention, la deuxième plaque, présente deux formes ondulées développées, une première forme ondulée développée épousant la forme du muscle psoas, et une deuxième forme ondulée développée épousant la forme des vaisseaux iliaques et spermatiques.

Dans un mode d'exécution préféré, les première et deuxième plaques sont constituées chacune d'un même matériau prothétique. Cependant, il s'est avéré avantageux que les première et deuxième plaques soient constituées chacune d'un matériau prothétique différent présentant une flexibilité différente. En effet, il a été trouvé particulièrement avantageux de prévoir, par exemple, que la première plaque, soit constituée d'un matériau prothétique relativement rigide, par exemple en un tricot ou un tissu simple épaisseur, et que la deuxième plaque, soit constituée d'un matériau prothétique relativement souple, par exemple en un tricot ou un tissu double épaisseur. De cette manière, la première plaque, supérieure, de la prothèse peut se tenir "debout" en quelque sorte, de manière sensiblement verticale, en reposant par le biais de la ligne de liaison, sur la ligne d'intersection des plans pariétaux et vasculaires.

Selon un mode d'exécution préféré, la première plaque a, dans la conformation déployée de la prothèse, une forme sensiblement en "L" renversé vers la droite, définissant une partie supérieure majoritaire en surface, et une partie inférieure sur le côté gauche, minoritaire en surface. Bien entendu, la première plaque peut avoir, de manière également préférée, dans la conformation déployée de la prothèse, une forme qui est l'image miroir de la forme sensiblement en "L" renversée vers droite précédemment décrite, définissant une partie supérieure majoritaire en surface, et une partie inférieure sur le côté droite, minoritaire en surface.

Par ailleurs, la première plaque, peut également présenter au moins une ondulation selon un bord transversal et le long de la ligne de liaison et complémentaire de l'ondulation de la deuxième plaque.

Le moyen de liaison, par exemple une couture mobile, permet aux deux plaques de se déplacer de manière limitée l'une par rapport à l'autre, afin de s'ajuster aux changements de position relative des éléments anatomiques de la région inguinale. Avantageusement, le moyen de liaison est obtenu par surfilage d'un bord transversal de chaque plaque.

Ainsi, l'angle d'ouverture θ entre la première plaque, et la deuxième plaque, est variable, en fonction de la position relative desdits éléments anatomiques, est au plus égal à 150°.

Selon un mode d'exécution préféré, la première plaque est fendue depuis un bord extérieur de celle-ci jusqu'à proximité du moyen de liaison.

Par ailleurs, et avantageusement, au moins une des première et deuxième plaques peut présenter une découpe adaptée à entourer le cordon spermatique. Dans un mode d'exécution selon la présente invention, les deux plaques présentent une découpe adaptée à entourer le cordon spermatique.

Plus préférentiellement encore, la première plaque est fendue et présente un feuillet avant muni d'un bord s'étendant de manière sensiblement perpendiculaire à la ligne de liaison depuis un bord extérieur de la première plaque vers une découpe, et un feuillet arrière muni d'un bord s'étendant de manière sensiblement oblique à la ligne de liaison depuis un bord extérieur de la première plaque vers ladite découpe, le feuillet avant recouvrant ainsi au moins partiellement le feuillet arrière.

Enfin, et de préférence, au moins une des première et deuxième plaques comporte des moyens de plicature ordonnée et structurée de la ou les plaques, par exemple un ou plusieurs fils entrelacés dans les mailles. Ceci a pour avantage de permettre au chirurgien de réduire la taille de la plaque ou des plaques de manière ordonnée, en tirant simplement sur les fils, pour pouvoir insérer la prothèse dans un trocart, et ensuite la déplier à nouveau à l'intérieur du corps de manière également ordonnée.

Dans une variante de l'invention, la prothèse anatomique est une prothèse bilatérale, constituée par un prolongement de la première plaque selon ses bords transversaux, et une troisième plaque, les formes et agencement du prolongement et de la troisième plaque étant sensiblement une image miroir de la première plaque et de la deuxième plaque respectivement, autour d'un axe de symétrie dans le même plan que ladite première plaque.

Conformément à l'ensemble de ces caractéristiques, on obtient une prothèse complètement anatomique, au point de pouvoir distinguer et fabriquer selon l'invention des prothèses gauche et droite. Cette prothèse est obtenue à partir de tous matériaux appropriés, dès lors qu'ils sont suffisamment poreux pour être colonisés par les cellules environnantes, et en quelque sorte intégrés, biocompatibles avec les tissus et milieux de l'espace inguinal, et mécaniquement résistants, notamment à la traction, le moyen de liaison étant au moins aussi résistant mécaniquement que la plus faible des deux plaques. A titre préférentiel, on préférera une étoffe (tissu et/ou tricot), ou un non tissé, obtenus à partir de fibres ou filaments naturels ou synthétiques, par exemple polypropylène ou polyester.

La présente invention sera mieux comprise par la description détaillée et le dessin suivants donnés à titre d'exemple non limitatif, dans lequel :
- **la figure 1** représente une vue en perspective du positionnement de la prothèse selon la présente invention par rapport aux éléments anatomiques de la région inguinale extrapéritonéale, du côté droit d'un corps humain, vue d'en dedans vers le dehors, c'est-à-dire vers l'extérieur du corps ;
- **la figure 2** représente une vue en plan des formes générales d'une première plaque, et d'une deuxième plaque, constituant la prothèse anatomique selon la présente invention ;
- **la figure 3** représente une vue en perspective des deux plaques de la prothèse selon l'invention, reliées l'une à l'autre par une couture mobile, et illustrant mieux les formes anatomiques de chaque plaque ;
- **la figure 4** représente une vue en coupe transversale de la prothèse selon l'invention selon la ligne IV-IV de la figure 3 ;
- **la figure 5** représente une variante de la prothèse selon l'invention, pour réparation de hernie bilatérale ;
- **la figure 6** représente un autre mode d'exécution préféré de la prothèse anatomique de base illustrée par la figure 1, avec de légères modifications ;
- **la figure 7** représente encore un autre mode d'exécution de la prothèse anatomique de base illustrée par la figure 1.

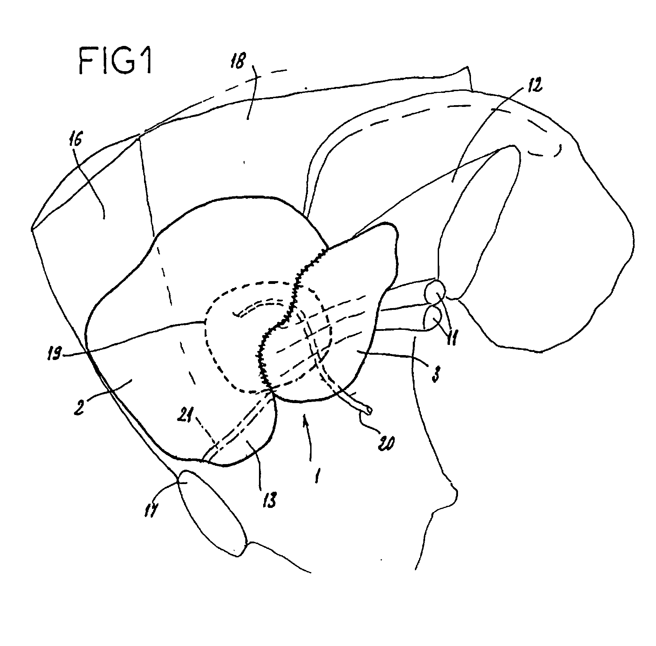

Conformément aux figures, et en particulier la figure 1, la prothèse selon la présente invention est utilisée pour réparer des hernies inguinales par voie postérieure laparoscopique ou ouverte. La technique utilisée dans l'intervention laparoscopique, par exemple, est bien connue à l'homme du métier et ne sera pas, par conséquent, décrite en détail. En résumant, un ou plusieurs trocarts sont introduits dans l'espace extrapéritonéal, c'est-à-dire postérieure au muscle grand droit et le fascia transversalis, l'espace de travail extrapéritonéal étant créé par insufflation et séparation du péritoine et de la paroi abdominale. La vue en perspective montrée en figure 1 représente donc une vue du positionnement de la prothèse selon la présente invention par rapport aux éléments anatomiques de la région inguinale extrapéritonéale, du côté droit d'un corps humain, vue d'en dedans vers le dehors, c'est-à-dire vers l'extérieur du corps. On y reconnaît les éléments anatomiques suivants de la paroi abdominale antérieure, de dedans en dehors, et pour ce côté droit du corps :
- en dedans, l'espace rétro-pariétal antérieur est limité en avant par le muscle grand droit 16, en arrière par le péritoine (non représenté), et en bas par le bord supérieur du pubis 17 ;
- la partie moyenne est limitée en avant par le fascia transversalis (non représenté), et le tendon conjoint, en bas on trouve les vaisseaux iliaques 11, et en haut le muscle transverse 18 ;
- dans la partie externe, on trouve en avant l'orifice interne 19 du canal inguinal avec les éléments du cordon (vaisseaux spermatiques), en bas le muscle psoas 12, et en haut le muscle transverse 18.

Dans cette figure, on voit clairement que la région inguinale est particulière en ce que les éléments décrits ci-dessus ne sont pas tous dans le même plan dans l'espace, mais disposés de manière oblique de haut en bas, et de dehors en dedans. En cas de hernie inguinale, la prothèse mise en place après réduction de la hernie doit assurer une bonne couverture en s'adaptant aux reliefs de la région et en respectant l'obliquité de l'espace inguinal. La figure 1 montre également le positionnement de la prothèse, et sa forme générale, dont les détails sont donnés ci-après.

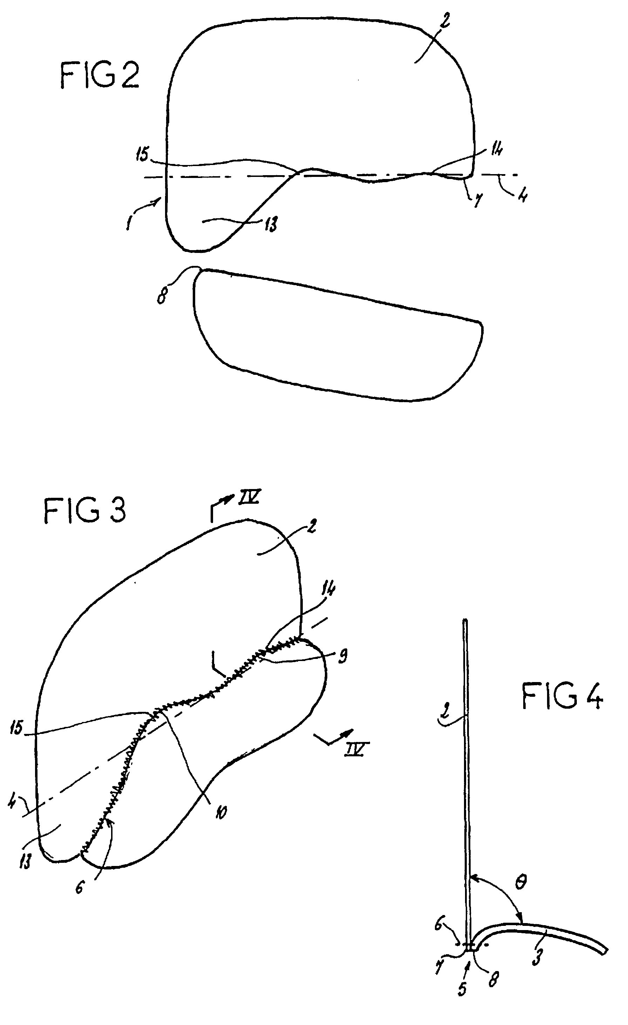

Dans un mode d'exécution préféré, et tel que représenté aux figures 2 à 4, la prothèse 1 selon l'invention comprend une première plaque 2, sensiblement plane, et une deuxième plaque 3, les deux plaques comprenant chacune, un matériau prothétique poreux et flexible, éventuellement enduit d'un agent favorisant la colonisation cellulaire, ou empêchant une telle colonisation cellulaire. Il est important de noter que les deux plaques sont asymétriques, l'une par rapport à l'autre, ce qui veut dire que selon le côté hernial à traiter, on utilisera une prothèse côté gauche ou une prothèse côté droit. Le matériau prothétique de la première plaque peut être différent, et/ou présente des propriétés mécaniques différentes du matériau de la deuxième plaque, les deux plaques pouvant présenter de ce fait une flexibilité différente.

Dans le mode d'exécution préféré, la première plaque 2 est constituée d'un matériau prothétique relativement rigide, par exemple en un tricot ou un tissu de simple épaisseur, et la deuxième plaque 3, est constituée d'un matériau prothétique relativement souple, par exemple en un tricot ou un tissu de double épaisseur, de préférence de l'ordre de 1,5 mm à 2 mm. Les deux plaques sont constituées de manière générale en tout matériau biocompatible, résorbable ou non, et de préférence en matière textile à base de fil polyester multifilaments. Une prothèse construite de cette façon permet de donner à la fois une certaine rigidité à la prothèse, facilitant sa manutention et mise en place par le chirurgien, ainsi qu'une souplesse permettant d'épouser toutes les formes anatomiques dans la région inguinale. Par ailleurs, une prothèse constituée de cette manière tend à "s'asseoir" en quelque sorte au fond de la région inguinale, sans se déplacer, ce qui était un problème connu des plaques prothétiques traditionnelles pour la réparation de hernies inguinales, et la première plaque se tient sensiblement "debout". De préférence, la première plaque 2 peut avoir une hauteur allant jusqu'à environ 15 cm, et la deuxième plaque 3 une profondeur comprise entre environ 2 cm et environ 6 cm. Les plaques peuvent en outre être optionnellement recouvertes d'une substance biologique, par exemple collagène, notamment du collagène bovin type I, ou également d'une substance polymère biocompatible.

Les plaques 2, 3 sont reliées entre elles selon une ligne de liaison 4, par un moyen de liaison, indiqué généralement par le numéro de référence 5. Dans le mode d'exécution montré dans les figures, par exemple les figures 3 et 4, mais de manière non limitative, ce moyen de liaison est une couture mobile 6. La figure 4, qui est une vue en coupe de la figure 3 selon les lignes IV-IV montre les deux plaques 2, 3 reliées par une telle couture, ainsi que les bords transversaux 7, 8 de chaque plaque. Cette couture est avantageusement obtenue par surfilage des plaques 2, 3, selon et au voisinage d'un bord transversal 7, 8 de chaque plaque (cf. figure 2). La couture mobile 6 permet un déplacement relatif des première 2 et deuxième 3 plaques, l'une par rapport à l'autre, ce qui augmente la capacité de la plaque à s'adapter aux mouvements musculaires de la personne chez qui elle est implantée. Il doit être entendu que le moyen de liaison 5 n'est pas limité à une telle couture 6, et que d'autres liaisons sont possibles, par exemple, par agrafage, tricotage, soudage ou collage des deux plaques, dans la mesure où la prothèse reste anatomique, et puisse épouser les formes anatomiques des structures inguinales sans laisser de vide sensible.

Dans la conformation déployée de la prothèse 1, c'est-à-dire implantée dans le corps, la deuxième plaque 3 présente au moins une forme développée ondulée, et anatomique pour épouser la forme générale des structures inguinales inférieures, à savoir les vaisseaux spermatiques et iliaques 11, et le muscle psoas 12. Selon le mode d'exécution préféré illustré par la figure 3, la première plaque 2 comporte deux ondulations 14, 15, une première ondulation 14 épousant la forme du muscle psoas 12, et une deuxième ondulation 15, épousant la forme des vaisseaux iliaques et spermatiques 11.

Dans ce cas, complémentaires des formes développées ondulées 9, 10 de la deuxième plaque 3, conformément à la figure 2, la ligne de liaison 4 présente également deux courbes ondulées en correspondance avec les formes développées ondulées de la deuxième plaque 3. Une génératrice décrit les formes ondulées développées 9, 10 et passe par la ligne de liaison 4, et est dirigée selon un angle d'ouverture au plus égal à 150°, par rapport au plan de la première plaque 2.

Comme on peut le voir sur les figures 2 à 4, et pour une prothèse de réparation de hernie du côté droit, la première plaque 2 présente avantageusement une forme sensiblement en "L" renversé vers la droite, définissant ainsi une partie supérieure majoritaire en surface, et une partie inférieure 13, sur le côté gauche, minoritaire en surface. Pour une prothèse côté gauche, on prendra l'image miroir de celle illustrée dans les figures 2 à 4, par exemple.

Lorsque la prothèse est implantée, et en se référant utilement à la figure 1, la partie majoritaire en surface de la première plaque 2 repose sur la paroi musculaire antérieure (notamment les muscles grands droits 16 et transversalis 18). La partie 13 minoritaire en surface de la première plaque 2 vient recouvrir l'extrémité supérieure du pubis et le ligament de Cooper 21 (représenté en traits mixtes). Les ondulations 9, 10 de la deuxième plaque 3 épousent sensiblement complètement et sans laisser de vides sensibles les vaisseaux iliaques et spermatiques 11, et le muscle psoas 12, et les bords transversaux 7, 8 reposent sur la ligne d'intersection des plans pariétaux et vasculaires. Le canal déférent 20, est également recouvert, contrairement aux prothèses de l'art antérieur, qui sont souvent munies de fentes ou perçages dans l'une des plaques, pour permettre le passage de ce canal, et pour lui servir "d'étrier", afin d'empêcher des récidives de hernies inguinales indirectes.

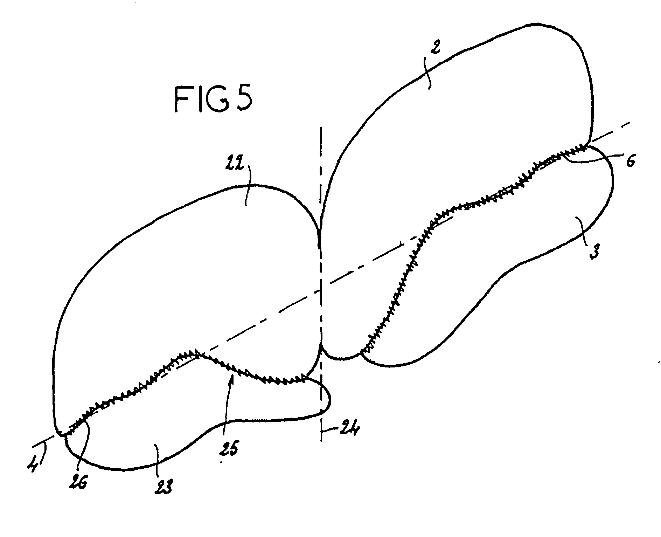

Dans une variante d'exécution de la présente invention, et comme illustrée par la figure 5, la prothèse est une prothèse bilatérale, servant à réparer une hernie bilatérale. Ainsi, la prothèse prend la forme d'une prothèse droite et gauche, telles que précédemment décrites, assemblées ou fabriquées de manière continue ou non. La première plaque 2 présente ainsi un prolongement 22 selon ses bords transversaux, et la prothèse comporte une troisième plaque 23, par rapport à la définition déjà donnée de celle-ci, les formes et agencement du prolongement 22 et de la troisième plaque 23 étant sensiblement une image miroir de la première plaque 2 et de la deuxième plaque 3 respectivement, autour d'un axe de symétrie 24 dans le même plan que ladite première plaque 2. La troisième plaque est reliée à la première plaque par le moyen de liaison 25, qui prend la forme d'une couture mobile 26 dans ce mode d'exécution de l'invention, et telle qu'illustrée par la figure 5. Cette prothèse peut se poser sensiblement de la même manière que pour le mode d'exécution préféré déjà présenté.

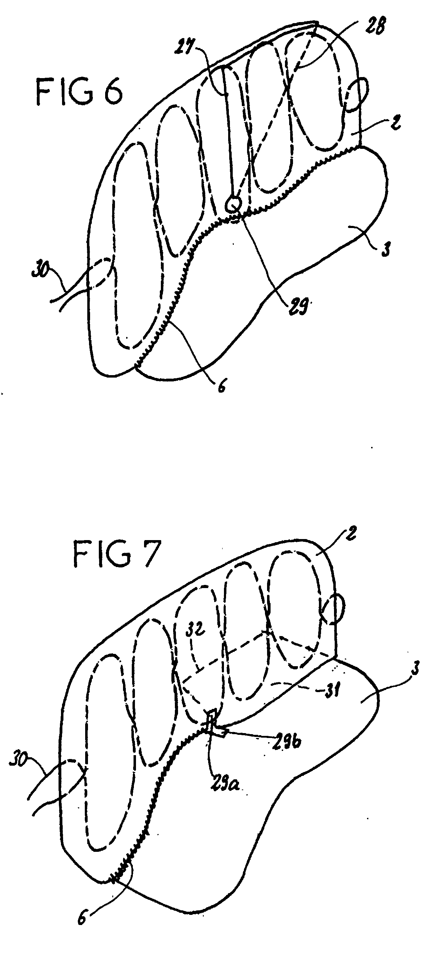

Les figures 6 et 7 représentent d'autres modes d'exécution préféré de la prothèse anatomique de base telle qu'illustrée par les figures 1 à 4. Selon la figure 6, la première plaque 2 de la prothèse est munie d'une découpe adaptée à entourer le cordon spermatique 11 et est fendue en deux feuillets 27, 28. Un feuillet avant 27 présente un bord s'étendant de manière sensiblement perpendiculaire à la ligne de liaison 4 depuis le bord extérieur de la première plaque 2 vers la découpe 29, et un feuillet arrière 28 présente un bord s'étendant de manière sensiblement oblique (indiqué en pointillés) à la ligne de liaison 4 depuis le bord extérieur de la première plaque 2 vers ladite découpe 29, le feuillet avant 27 recouvrant ainsi au moins partiellement le feuillet arrière 28. Cette structure permet d'introduire la prothèse et la disposer autour des vaisseaux spermatiques, tout en offrant une couverture suffisante de la région abdominale antérieure, et permet en outre de s'affranchir de la fermeture de la fente par suture ou agrafage. La prothèse illustrée par la figure 6 dispose également de moyens de plicature 30 ordonnée de la prothèse, permettant de rassembler celle-ci de manière structurée afin de faciliter son introduction dans un trocart, ainsi que son déploiement à l'intérieur du corps. Ces moyens de plicature peuvent être, par exemple, des fils chirurgicaux entrelacés entre les mailles de la plaque ou des plaques de la prothèse.

La prothèse illustrée par la figure 7 sera décrite uniquement par rapport à ses différences avec celle de la figure 6. Ainsi la première plaque 2 n'est pas fendue, mais en revanche une fente sensiblement horizontale est créée entre les deux plaques du fait qu'elles ne sont reliées l'une à l'autre que sur une partie de leurs bords transversaux. En outre chaque plaque 2, 3 comporte une découpe 29a, 29b adaptée à entourer le cordon spermatique, et la deuxième plaque 3 présente un prolongement 31 à bord sensiblement droit. En effet, dans la position déployée de la prothèse, ce prolongement 31 passe en dessous du bord inférieur de la première plaque 2, et est replié derrière celle-ci par la paroi abdominale de manière à former un angle aigu avec le reste de la deuxième plaque.

Les avantages de la prothèse selon la présente invention sont nombreuses, car l'adéquation de sa forme à l'anatomie rétropéritonéale de la région inguinale facilite son positionnement et réduit par conséquent le temps nécessaire à son placement. Par ailleurs, lorsque le matériau constitutif de la deuxième plaque est souple, par exemple en tissu tridimensionnel, cette souplesse ainsi que la porosité du matériau facilite le moulage de la prothèse sur les reliefs ventraux.

Ces avantages ont été démontrés lors d'un suivi clinique mené sur des patients humains avec une prothèse non fendue selon l'invention dans deux centres indépendants, et donnent les résultats suivants :

### Nombre de hernies traitées :

- 252 avec prothèses fixées (un point sur le ligament de Cooper et un point sur la paroi abdominale) ;
- 51 avec prothèses non fixées.

### Voie d'abord utilisée :

- laparoscopie extrapéritonéale.

### Recul moyen :

- six mois pour le groupe des prothèses fixées ;
- quatre mois pour le groupe des prothèses non fixées.

### Durée de suivi minimum/maximum :

- un mois/douze mois.

| Résultats | |
|---|---|
| Migration précoce (totale des deux groupes) | 0 |
| Récidives | 0 |
| Sepsis | 0 |
| Complications attribuables à la forme de la prothèse | 0 |

## Revendications

1. Prothèse anatomique (1) adaptée spécialement pour la région inguinale et destinée à la réparation de hernies inguinales par voie laparoscopique postérieure ou ouverte, la prothèse (1) comprenant une première plaque (2), sensiblement plane, et une deuxième plaque (3), lesdites plaques comprenant chacune un matériau prothétique poreux et flexible, et étant reliées entre elles selon une ligne de liaison (4) par un moyen de liaison (5), notamment une couture, **caractérisée en ce que** les deux plaques sont asymétriques l'une par rapport à l'autre, et dans la conformation déployée de la prothèse, la deuxième plaque (3) présente au moins une forme développée ondulée (9), et anatomique pour épouser la forme générale des structures inguinales inférieures, notamment les vaisseaux spermatiques et iliaques (11), et le muscle psoas (12), et en correspondance la ligne de liaison (4) présente au moins une forme courbe ondulée, la génératrice décrivant ladite forme développée et passant par la ligne de liaison (4) étant dirigée selon un angle d'ouverture θ au plus égal à 150° par rapport au plan de la première plaque.

2. Prothèse anatomique (1) selon la revendication 1, **caractérisée en ce que** la deuxième plaque (3), présente deux formes ondulées développées (9, 10), une première forme ondulée développée (9) épousant la forme du muscle psoas (12), et une deuxième forme ondulée développée (10) épousant la forme des vaisseaux iliaques et spermatiques (11).

3. Prothèse anatomique (1) selon la revendication 1, **caractérisée en ce que** les première (2) et deuxième (3) plaques sont constituées chacune d'un même matériau prothétique

4. Prothèse anatomique (1) selon la revendication 1, **caractérisée en ce que** les première (2) et deuxième (3) plaques sont constituées chacune d'un matériau prothétique présentant une flexibilité différente.

5. Prothèse anatomique (1) selon la revendication 1, **caractérisée en ce que** la première plaque (2), est constituée d'un matériau prothétique relativement rigide, par exemple en un tricot ou un tissu de simple épaisseur, et la deuxième plaque (3), est constituée d'un matériau prothétique relativement souple, par exemple en un tricot ou un tissu double épaisseur.

6. Prothèse selon la revendication 1, **caractérisée en ce que** la première plaque (2) a, dans la conformation déployée de la prothèse, une forme sensiblement en "L" renversé vers la droite, définissant une partie supérieure majoritaire en surface, et une partie inférieure (13) sur le côté gauche, minoritaire en surface.

7. Prothèse selon la revendication 1, **caractérisée en ce que** la première plaque (2) a, dans la conformation déployée de la prothèse, une forme qui est l'image miroir d'une forme sensiblement en "L" renversée vers la droite, définissant une partie supérieure majoritaire en surface, et une partie inférieure sur le côté droit, minoritaire en surface.

8. Prothèse anatomique (1) selon la revendication 1, **caractérisée en ce que** le moyen de liaison (5) est obtenu par surfilage d'un bord transversal (7, 8) de chaque plaque (2, 3).

9. Prothèse anatomique (1) selon la revendication 1, **caractérisée en ce qu'**elle est une prothèse anatomique bilatérale, constituée par un prolongement (22) de la première plaque (2) selon ses bords transversaux, et une troisième plaque (23), les formes et agencement du prolongement (22) et de la troisième plaque (23) étant sensiblement une image miroir de la première plaque (2) et de la deuxième plaque (3) respectivement, autour d'un axe de symétrie (24) dans le même plan que ladite première plaque (2).

10. Prothèse anatomique (1) selon la revendication 1, **caractérisée en ce que** la première plaque (2) est fendue depuis un bord extérieur de celle-ci jusqu'à proximité du moyen de liaison (5).

11. Prothèse anatomique (1) selon la revendication 1, **caractérisée en ce qu'**au moins une des première et deuxième plaques présente une découpe (29) adaptée à entourer le cordon spermatique.

12. Prothèse anatomique (1) selon la revendication 11, **caractérisée en ce que** les deux plaques présentent une découpe (29a,29b) adaptée à entourer le cordon spermatique.

13. Prothèse anatomique (1) selon la revendication 1, **caractérisée en ce que** la première plaque (2) est fendue et présente un feuillet avant (27) muni d'un bord s'étendant de manière sensiblement perpendiculaire à la ligne de liaison (4) depuis un bord extérieur de la première plaque (2) vers une découpe (29), et un feuillet arrière (28) muni d'un bord s'étendant de manière sensiblement oblique à la ligne de liaison (4) depuis un bord extérieur de la première plaque (2) vers ladite découpe (29), le feuillet avant (27) recouvrant ainsi au moins partiellement le feuillet arrière (28).

14. Prothèse anatomique (1) selon la revendication 1, **caractérisée en ce que** au moins une des première et deuxième plaques comporte des moyens de plicature (30) ordonnée et structurée de la ou les plaques.

## Claims

1. Anatomical prosthesis (1) adapted specially for the inguinal region and intended for the repair of inguinal hernias by a posterior laparoscopic or open route, the prosthesis (1) comprising a first plate (2), which is substantially plane, and a second plate (3), the said plates each comprising a porous and flexible prosthetic material, and being connected to one another along a connection line (4) by a connection means (5), especially a seam, **characterized in that** the two plates are asymmetrical in relation to one another, and, in the deployed configuration of the prosthesis, the second plate (3) has at least one undulated developed shape (9), and anatomical so as to match the general shape of the lower inguinal structures, especially the spermatic and iliac vessels (11), and the psoas muscle (12), and correspondingly the connection line (4) has at least one undulated curved shape, the generatrix describing the said developed shape and passing through the connection line (4) being directed at an aperture angle θ at most equal to 150° relative to the plane of the first plate.

2. Anatomical prosthesis (1) according to Claim 1, **characterized in that** the second plate (3) has two developed undulated shapes (9, 10), a first developed undulated shape (9) matching the shape of the psoas muscle (12), and a second developed undulated shape (10) matching the shape of the iliac and spermatic vessels (11).

3. Anatomical prosthesis (1) according to Claim 1, **characterized in that** the first (2) and second (3) plates are each made of the same prosthetic material.

4. Anatomical prosthesis (1) according to Claim 1, **characterized in that** the first (2) and second (3) plates are each made of a prosthetic material exhibiting a different flexibility.

5. Anatomical prosthesis (1) according to Claim 1, **characterized in that** the first plate (2) is made of a relatively rigid prosthetic material, for example of a knitted fabric or a woven fabric of single thickness, and the second plate (3) is made of a relatively flexible prosthetic material, for example a knitted fabric or a woven fabric of double thickness.

6. Prosthesis according to Claim 1, **characterized in that** the first plate (2) has, in the deployed configuration of the prosthesis, a substantially L-shaped form tilted over to the right, defining an upper part of greater surface area, and a lower part (13), on the left-hand side, of smaller surface area.

7. Prosthesis according to Claim 1, **characterized in that** the first plate (2) has, in the deployed configuration of the prosthesis, a shape which is the mirror image of a substantially L-shaped form tilted over to the right, defining an upper part of greater surface area, and a lower part, on the right-hand side, of smaller surface area.

8. Anatomical prosthesis (1) according to Claim 1, **characterized in that** the connection means (5) is obtained by overstitching a transverse edge (7, 8) of each plate (2, 3).

9. Anatomical prosthesis (1) according to Claim 1, **characterized in that** it is a bilateral anatomical prosthesis, formed by a continuation (22) of the first plate (2) along its transverse edges, and a third plate (23), the shapes and arrangement of the continuation (22) and of the third plate (23) being substantially a mirror image of the first plate (2) and of the second plate (3), respectively, about an axis of symmetry (24) in the same plane as the said first plate (2).

10. Anatomical prosthesis (1) according to Claim 1, **characterized in that** the first plate (2) is slotted from an outer edge thereof up to a position near the connection means (5).

11. Anatomical prosthesis (1) according to Claim 1, **characterized in that** at least one of the first and second plates has a cutout (29) adapted to surround the spermatic cord.

12. Anatomical prosthesis (1) according to Claim 11, **characterized in that** both plates have a cutout (29a, 29b) adapted to surround the spermatic cord.

13. Anatomical prosthesis (1) according to Claim 1, **characterized in that** the first plate (2) is slotted and has a front leaf (27) equipped with an edge extending, substantially perpendicular to the connection line (4), from an outer edge of the first plate (2) towards a cutout (29), and a rear leaf (28) equipped with an edge extending, substantially oblique to the connection line (4), from an outer edge of the first plate (2) towards the said cutout (29), the front leaf (27) thereby at least partially covering the rear leaf (28).

14. Anatomical prosthesis (1) according to Claim 1, **characterized in that** at least one of the first and second plates includes means (30) for ordered and structured folding of the plate or plates.

## Patentansprüche

1. Anatomische Prothese (1), die speziell für die Leistengegend ausgelegt und für die Reparation von Leistenbrüchen auf laparoskopischem posteriorem oder offenem Wege bestimmt ist, wobei die Prothese (1) eine etwa ebene erste Platte (2) und eine zweite Platte (3) aufweist, wobei die Platten jeweils ein poröses und flexibles prothetisches Material aufweisen und untereinander entlang einer Verbindungslinie (4) durch ein Verbindungsmittel (5), insbesondere eine Naht, verbunden sind, **dadurch gekennzeichnet, dass** die beiden Platten in Bezug aufeinander asymmetrisch sind und die zweite'Platte (3) in dem ausgebreiteten Zustand der Prothese zumindest eine abgewickelte gewellte Form (9) aufweist, und die anatomisch ist, um sich an die allgemeine Form der inferioren Leistenstrukturen, insbesondere der Samen- und Darmbeingefäße (11) und den Lendenmuskel (12) anzupassen, und dass in Entsprechung die Verbindungslinie (4) zumindest eine gekrümmte gewellte Form aufweist, wobei die Erzeugende, die die abgewickelte Form beschreibt und durch die Verbindungslinie (4) durchgeht, unter einem Öffnungswinkel θ von höchstens gleich 150° bezüglich der Ebene der ersten Platte gerichtet ist.

2. Anatomische Prothese (1) nach Anspruch 1, **dadurch gekennzeichnet, dass** die zweite Platte (3) zwei abgewickelte gewellte Formen (9, 10) aufweist, wobei eine erste abgewickelte gewellte Form (9) sich an die Form des Lendenmuskels (12) und eine zweite abgewickelte gewellte Form (10) an die Form der Darmbein- und Samengefäße (11) anpasst.

3. Anatomische Prothese (1) nach Anspruch 1, **dadurch gekennzeichnet, dass** die erste (2) und zweite (3) Platte jeweils aus einem gleichen prothetischen Material gebildet sind.

4. Anatomische Prothese (1) nach Anspruch 1, **dadurch gekennzeichnet, dass** die erste (2) und zweite (3) Platte jeweils aus einem prothetischen Material gebildet sind, das eine unterschiedliche Flexibilität aufweist.

5. Anatomische Prothese (1) nach Anspruch 1, **dadurch gekennzeichnet, dass** die erste Platte (2) aus einem relativ steifen prothetischen Material, beispielsweise aus einem Gewirk oder einem Gewebe von einfacher Dicke gebildet ist, und dass die zweite Platte (3) aus einem relativ biegsamen prothetischen Material, beispielsweise einem Gewirk oder einem Gewebe doppelter Dicke gebildet ist.

6. Prothese nach Anspruch 1, **dadurch gekennzeichnet, dass** die erste Platte (2) in dem ausgebreiteten Zustand der Prothese etwa eine Form eines nach rechts umgekehrten "L" aufweist, das einen bezüglich der Oberfläche größeren oberen Bereich und auf der linken Seite einen bezüglich der Oberfläche kleineren unteren Bereich (13) definiert.

7. Prothese nach Anspruch 1, **dadurch gekennzeichnet, dass** die erste Platte (2) in dem ausgebreiteten Zustand der Prothese eine Form aufweist, die das Spiegelbild einer Form etwa eines nach rechts umgekehrten "L" ist, die einen bezüglich der Oberfläche größeren oberen Bereich und einen auf der rechten Seite bezüglich der Oberfläche kleineren unteren Bereich definiert.

8. Anatomische Prothese (1) nach Anspruch 1, **dadurch gekennzeichnet, dass** das Verbindungsmittel (5) durch Umstechen eines quer verlaufenden Randes (7, 8) jeder Platte (2, 3) erhalten ist.

9. Anatomische Prothese (1) nach Anspruch 1, **dadurch gekennzeichnet, dass** sie eine zweiseitige anatomische Prothese ist, die durch eine Verlängerung (22) der ersten Platte (2) entlang seiner quer verlaufenden Ränder und eine dritte Platte (23) gebildet ist, wobei die Formen und der Schnitt der Verlängerung (22) der dritten Platte (23) etwa ein Spiegelbild der ersten Platte (2) bzw. der zweiten Platte (3) um eine Symmetrieachse (24) in der gleichen Ebene wie die erste Platte (2) sind.

10. Anatomische Prothese (1) nach Anspruch 1, **dadurch gekennzeichnet, dass** die erste Platte (2) von einem äußeren Rand derselben bis in die Nähe des Verbindungsmittels (5) geschlitzt ist.

11. Anatomische Prothese (1) nach Anspruch 1, **dadurch gekennzeichnet, dass** zumindest eine der ersten und zweiten Platte einen Ausschnitt (29) aufweist, der dazu ausgelegt ist, den Samenstrang zu umgeben.

12. Anatomische Prothese (1) nach Anspruch 11, **dadurch gekennzeichnet, dass** die beiden Platten einen Ausschnitt (29a, 29b) aufweisen, der dazu ausgelegt ist, den Samenstang zu umgeben.

13. Anatomische Prothese (1) nach Anspruch 1, **dadurch gekennzeichnet, dass** die erste Platte (2) geschlitzt ist und eine vordere Lamelle (27), die mit einem Rand versehen ist, der sich etwa senkrecht zur Verbindungslinie (4) von einem äußeren Rand der ersten Platte (2) zu einem Ausschnitt (29) erstreckt, und eine hintere Lamelle (28) aufweist, die mit einem Rand versehen ist, der sich etwa schräg zur Verbindungslinie (4) von einem äußeren Rand der ersten Platte (2) zu dem Ausschnitt (29) erstreckt, wobei die vordere Lamelle (27) so zumindest teilweise die hintere Lamelle (28) überdeckt.

14. Anatomische Prothese (1) nach Anspruch 1, **dadurch gekennzeichnet, dass** zumindest eine der ersten und zweiten Platte Plikaturmittel (30) zur geordneten und strukturierten Plikatur der einen oder beider Platten aufweist.
